# EUROPÄISCHE PATENTSCHRIFT

(11) **EP 0 967 832 B1**
(45) Veröffentlichungstag und Bekanntmachung des Hinweises auf die Patenterteilung: **31.07.2013**
(21) Anmeldenummer: 99112047.8
(22) Anmeldetag: 22.06.1999
(51) Int. Cl.: A61F 2/18, A61N 1/24, A61N 1/28, A61N 1/378, H01L 35/00, H01L 35/32

(54) **Verfahren und Vorrichtung zur Versorgung eines mindestens teilimplantierten aktiven Gerätes mit elektrischer Energie**
Method and device for supplying electric energy to a partially implanted active device
Méthode et dispositif pour la délivrance d'énergie électrique à un dispositif actif partiellement implanté

(30) Priorität: 23.06.1998 DE 19827898
(43) Veröffentlichungstag der Anmeldung: 29.12.1999
(73) Patentinhaber: Cochlear Limited, Lane Cove, NSW 2066 (AU)
(72) Erfinder: Leysieffer, Hans, Dr.Ing., 82024 Taufkirchen (DE); Müller, Gerd M., Dr.rer.nat., 85716 Unterschleissheim (DE)
(74) Vertreter: Grünecker, Kinkeldey, Stockmair & Schwanhäusser

(56) Entgegenhaltungen:
- DE-A1- 19 530 382
- US-A- 2 798 493
- US-A- 4 213 292
- PATENT ABSTRACTS OF JAPAN Bd. 1996, Nr. 06, 28. Juni 1996 (1996-06-28) & JP 08 029559 A (SEIKO INSTR INC), 2. Februar 1996 (1996-02-02)

## Beschreibung

Die Erfindung betrifft eine Vorrichtung zur Versorgung eines mindestens teilimplantierten aktiven Gerätes, insbesondere einer Hörhilfe, mit elektrischer Energie, die von einem implantierten, auf dem Seebeck - Effekt basierenden thermoelektrischen Energiewandler erzeugt wird.

Ein solche Vorrichtung wurden allgemein schon von H.-J. Wanjura in dem Aufsatz "Zur künftigen Energieversorgung von Herzschrittmachern" in Medizinal-Markt/Acta Medicotechnica, 1969, Nr. 3, S. 98-100 vorgeschlagen. Ein konkrete Ausführung ist aus DE 195 30 382 A1 bekannt. Dabei wird der menschliche Körper als Wärmequelle für einen Energiewandler genutzt, der in dem dort als bevorzugt offenbarten Ausführungsbeispiel aus einem Zinkdraht und einem Kupferdraht besteht. Die Verbindungsstelle der beiden Drähte wird der Wärmequelle ausgesetzt, bei der es sich vorzugsweise um die menschliche Haut handelt. Eine unregelmäßige Spannungserzeugung durch den Energiewandler wird von einem Kondensator abgepuffert. Die bereitgestellte Spannung kann einem Mikroprozessor zugeführt werden, der für eine konstante Gleichspannung sorgt.

Die Nutzung des Seebeck-Effektes zur Bereitstellung der zum Betrieb von mindestens teilweise implantierbaren aktiven Systemen benötigten elektrischen Energie hat bislang keinen Eingang in die medizinische Praxis gefunden. Dies dürfte darauf zurückzuführen sein, daß die mit den bekannten Vorrichtungen erzielbaren Energiewerte unzureichend sind, unter anderem weil die an dem thermoelektrischen Energiewandler verfügbaren Temperaturdifferenzen jedenfalls dann sehr klein sind, wenn der Energiewandler implantiert wird.

Die US 3,969,149 betrifft einen thermoelektrischen Mikrogenerator und offenbart alle Merkmale des einleitenden Teils des Anspruches 1.

Die DE 19530382 A betrifft ein Verfahren und eine Vorrichtung zum Erzeugen von elektrischem Strom und die Verwendung des Verfahrens und der Vorrichtung. Insbesondere, beschreibt dieses Dokument einen thermoelektrischen Energiewandler, der den Seebeck Effekt nutzt.

Der Erfindung liegt die Aufgabe zugrunde, eine Vorrichtung zur Versorgung eines teil- oder vollimplantierten aktiven Gerätes, insbesondere einer Hörhilfe, mit elektrischer Energie zu schaffen, wobei die Energie von einem implantierten, auf dem Seebeck - Effekt basierenden thermoelektrischen Energiewandler erzeugt wird.

Ausgehend von einer Vorrichtung der eingangs genannten Art wird diese Aufgabe erfindungsgemäß gemäß des Gegenstandes des Patentanspruches 1 gelöst, wober der Wärmepol des Energiewandlers in einem Bereich der Körperoberfläche, vorzugsweise im Schädel- oder Halsbereich, des Implantatträgers so positioniert wird, daß er, bevorzugt durch Wärmekopplung mit einer der großen Venen oder Arterien, mindestens näherungsweise auf Körperkerntemperatur gehalten wird, und daß der Kältepol des Energiewandlers mit einem möglichst nahe der Körperoberfläche in Außenhautnähe liegenden Bereich, vorzugsweise einem möglichst nahe der Körperoberfläche liegenden Schädel- oder Halsbereich, des Implantatträgers in Wärmekopplung gehalten wird.

Eine Vorrichtung zur Versorgung eines mindestens teilimplantierten aktiven Gerätes, insbesondere einer Hörhilfe, mit elektrischer Energie, mit einem implantierbaren, auf dem Seebeck - Effekt basierenden thermoelektrischen Energiewandler, der vom Körper des Implantatträgers bereitgestellte thermische Energie nutzt, ist erfindungsgemäß dadurch gekennzeichnet, daß der Energiewandler eine Mehrzahl von miteinander elektrisch gekoppelten Einzelmodulen aufweist, daß der Wärmepol des Energiewandlers zur Wärmekopplung mit einem mindestens näherungsweise auf Körperkerntemperatur gehaltenen Implantationsort im Bereich der Körperoberfläche, insbesondere im Schädel- oder Halsbereich, vorzugsweise einer der großen Venen oder Arterien, des Implantatträgers ausgebildet ist und daß der Kältepol des Energiewandlers zur Wärmekopplung mit einem möglichst nahe der Körperoberfläche in Außenhautnähe liegenden Bereich, vorzugsweise einem möglichst nahe der Körperoberfläche liegenden Schädel- oder Halsbereich, des Implantatträgers ausgebildet ist.

Bei der Vorrichtung nach der Erfindung wird die ständig zur Verfügung stehende thermische Energie des Körpers direkt in elektrische Energie umgewandelt.

Dies geschieht dadurch, daß die konstruktionsbedingte und durch den Implantationsort gegebene Temperaturdifferenz zwischen Körperkerntemperatur und Körperoberflächentemperatur genutzt wird. Diese Temperaturdifferenz als thermodynamische Energieform wird durch den physikalischen Seebeck-Effekt direkt in elektrische Energie umgewandelt. Der Seebeck-Effekt ist die Umkehrform des bekannten Peltier-Effektes, bei dem durch Zufuhr von elektrischer Energie in eine geeignete Werkstoffpaarung eine Temperaturdifferenz an örtlich verschiedenen Zonen des Elementes erzeugt wird. Der Seebeck-Effekt wird erreicht z.B. durch die konstruktive Paarung von Metallen, die in der thermoelektrischen Spannungsreihe einen möglichst weiten Abstand aufweisen. Eine noch wirkungsvollere Ausnutzung des Effektes wird durch die konstruktive Verbindung von speziell dotierten Halbleitermaterialien erzielt.

Der Schädel- oder Halsbereich des Implantatträgers ist nur selten oder nie von wärmeisolierender Kleidung bedeckt. Er hat daher normalerweise eine Temperatur, die unter der Körperkerntemperatur liegt. Andererseits sind die in diesem Bereich befindlichen großen Venen oder Arterien, zum Beispiel die Vena jugularis externa, die Vena jugularis anterior, der Sinus transversus oder die Arteria carotis externa, blutdurchströmte Gefäße, welche einen kontinuierlichen Wärmeenergielieferant mit kleinem thermischen Innenwiderstand darstellen, der auch bei andauerndem Wärmeentzug durch das Wandlerelement permanent ausreichend thermische Körperenergie nachliefern kann. Die erfindungsgemäße Art der Wärmeankopplung für den Kältepol und den Wärmepol stellt daher eine für den praktischen Einsatz ausreichende Temperaturdifferenz sicher. Es kann dabei von einem im zeitlichen Mittel konstanten ΔT ausgegangen werden, so daß sich durch das Seebeck-Element zeitlich unbegrenzt, langzeitstabil und verschleißfrei direkt elektrische Energie gewinnen läßt. Wesentliche Vorteile gegenüber bisher verwendeten implantierbaren Energieversorgungssystemen, wie primäre oder sekundäre elektrochemische Elemente (DE 4 104 359 C, US-PS 4 134 408, EP 0 341 902 A), Biobrennstoffzellen (DE-OS 2 200 054 und DE-OS 2 415 385), Nuklearbatterien (EP 0 761 256 A) und Vorrichtungen zur direkten Wandlung von mechanischer Bewegungsenergie (z. B. des Herzmuskels) in elektrische Energie mittels mechanoelektrischer Wandlerprinzipien [Piezoeffekt (DE-OS 2 729 223), mechanische Unruhe-Systeme und dergleichen(DE 1 764 622 C, DE 2 020 380 C], bestehen darin, daß
- das Seebeck-Element nicht selbstverbrauchend arbeitet, d.h., es werden keine Stoffe umgesetzt;
- das Seebeck-Element keinerlei mechanisch bewegten Teile aufweist und daher keinem mechanischen Verschleiß unterliegt;
- die Lebensdauer aus den vorstehend genannten Gründen prinzipiell unbegrenzt ist;
- der Implantatträger sich nicht bewußt um die energetische Versorgung seines Implantates bemühen muß, d.h. das Implantatsystem geht für lange Zeit in einen "unterbewußten Zustand" ein;
- die Energiezufuhr durch die körperintern geregelte Temperatur eingangsseitig weitgehend konstant ist.

Grundlegende eigene Laborversuche mit handelsüblichen Seebeck-Elementen haben gezeigt:
- Bei einer angenommenen Temperaturdifferenz von 0,5 K, einer Querschnittsfläche von ca. 0,25 mm² und einem Volumen eines Elementes unter 1 mm³ ist eine elektrische Ausgangsleistung von rund 0,5 µWatt erreichbar.
- Bei nur zehn dieser Elemente ergibt sich eine elektrische Dauerleistung von etwa 5 µWatt. Diese Leistung ist bereits ausreichend, um die Stand-By-Leistungsaufnahme eines modernen Herzschrittmachers oder Neurostimulators zu decken, solange kein neurostimulierender Reizimpuls abgesetzt wird.
- Durch geeignete Anordnung von einer großen Zahl (im Bereich über 100.000) miniaturisierter Seebeck-Elemente durch z.B. mikrosystemtechnische oder halbleiter-lithografische Verfahren kann bei geeigneter Verschaltung der Einzelelemente ein Gesamtsystem realisiert werden, das bei einer abschätzbaren Modulfläche im Bereich von 100 bis 300 cm² und einer Temperaturdifferenz von 0,5 K kontinuierlich elektrische Leistungen im Bereich von 1 bis 3 mWatt bei einer Klemmenspannung von 1 bis 2 Volt abgeben kann. Die elektrische Quellimpedanz (Innenwiderstand) liegt dabei in einem Bereich von 100 bis 300 Ω, so daß ein kontinuierlich arbeitendes Implantat, wie insbesondere Hörhilfen, energetisch versorgbar ist.

In bevorzugter weiterer Ausgestaltung der Erfindung wird der Wärmepol des Energiewandlers mit dem Sinus Sigmoideus des Implantatträgers in Wärmekopplung gehalten wird, während der Kältepol des Energiewandlers mit der benachbart dem Sinus Sigmoideus nahe der Körperoberfläche liegenden Mastoidregion des Implantatträgers hinter dem Außenohr in Wärmekopplung gehalten wird. Dort steht Knochenstruktur in ausreichendem Maße nahe unter der Hautoberfläche zur Verfügung, und die den Sinus Sigmoideus bedekkende knöcherne Struktur ist nach einer operationstechnischen Standardprozedur (Mastoidektomie) relativ einfach zugänglich. Es ist eine weitgehend konstante Nachlieferung von Wärmeenergie an den Wärmepol gewährleistet.

Von dem Energiewandler erzeugte elektrische Energie kann in einer implantierbaren Speicheranordnung gesammelt und zwischengespeichert werden, bis sie von dem mindestens teilimplantierten aktiven Gerät benötigt wird. Ein typisches Anwendungsbeispiel sind "On-demand-"Herzschrittmacher, die die meiste Zeit im "Stand-By-Modus" arbeiten und dort nur wenige µWatt benötigen; im Bedarfsfall wird ein kurzer, hochenergetischer Reizimpuls im mWatt-Bereich zur Herzstimulation erzeugt. Auch andere Neurostimulatoren können so versorgt werden, die in nur unregelmäßigen und größeren zeitlichen Abständen höhere Betriebsenergie für kurze Reizimpulse benötigen. Bei entsprechend vorgesehenen, nachgeschalteten elektrischen Speichermedien wie langlebigen Kondensatoren (Gold - Cap, OSCON - Kondensatoren oder hochqualitative Festelektrolyt - Tantal - Kondensatoren) und elektronischen Energie-Managementsystemen kann eine kurzzeitige Leistungsbedarfsspitze für eine Neurostimulation im Leistungsbereich einiger mWatt abgesetzt werden, wenn diese Reizimpulse im größeren zeitlichen Abstand erfolgen. Weiterhin kann mit Kleinstsystemen von bereits wenigen Seebeck-Elementen eine sekundäre Batteriezelle (Akkumulator) geladen werden, die dann die temporär verteilte Bedarfsenergie zur Verfügung stellt.

Die Zwischenspeicherung der von dem Energiewandler erzeugten elektrische Energie in der implantierbaren Speicheranordnung und/oder die Energieweiterleitung an den Verbraucher können zweckmäßig über eine implantierbare Steuereinheit, wie einen Mikroprozessor oder Mikrokontroller, geregelt werden, der entsprechende Peripherie-Hardware (AD- und DA-Wandler, Stromstellglieder und dergleichen) zugeordnet ist und die ihrerseits nur sehr wenig elektrische Betriebsleistung erfordert, da in der Regel keine zeitlich schnellen Vorgänge bearbeitet werden müssen.

Die implantierbare Steuereinheit kann insbesondere über eine Datenschnittstelle drahtlos, transkutan von außerhalb des Körpers des Implantatträgers über eine entsprechende externe Steueranordnung programmierbar sein, um das Gesamtsystem "Programmierbarer thermoelektrischer Energiewandler" an das im speziellen Anwendungs- und Kombinationsfall gewählte aktive Implantat als Verbraucher anpassen zu können.

Der Energiewandler weist bevorzugt eine Vielzahl von miteinander elektrisch gekoppelten Einzelmodulen auf, wobei jedes Einzelmodul mit zwei Schenkeln aus unterschiedlich elektrisch leitenden Werkstoffen versehen ist, deren eines Ende mit dem Kältepol und deren anderes Ende mit dem Wärmepol des Energiewandlers wärmeleitend verbunden ist. Der Aufbau des Energiewandlers und dessen Herstellungsverfahren können von an sich bekannten Merkmalen Gebrauch machen, wie sie unteren anderem aus US-PS 4 095 998, US-PS 5 439 528, US-PS 5 430 322, JP 09 092 889 A, EP 0 731 513, WO 94/16464, WO 96/15412 und WO 97/44993 bekannt sind.

Die Schenkel der Einzelmodule können insbesondere aus Halbleiterwerkstoffen mit n-beziehungsweise p-Dotierung oder aus unterschiedlichen Metallen mit großem Abstand in der thermoelektrischen Spannungsreihe bestehen. Als Materialkombinationen für das bzw. die Seebeck-Elemente kommen unter anderem Germanium-Silizium-Mischkristalle und Wismut-Tellur-Kombinationen als Halbleiterelemente mit geeignet gewählter Dotierung in Frage. Weitere grundsätzlich geeignete Werkstoffkombinationen sind unter anderem in WO 94/14200, EP 0 712 537 A sowie in US-PS 5 747 728 und US-PS 5 356 485 sowie den dort aufgelisteten Literaturstellen beschrieben.

Der Kältepol und der Wärmepol sind vorteilhaft aus biokompatiblem, gut wärmeleitendem Werkstoff gefertigt. Dabei kommen insbesondere reines Titan, Titanlegierungen, Niob, Nioblegierungen, Tantal, Tantallegierungen, Edelstähle und keramische Werkstoffe wie Aluminiumoxid-Keramik in Betracht.

Die Einzelmodule sind zweckmäßig durch einen zwischen dem Kältepol und dem Wärmepol angeordneten biokompatiblen thermischen Isolator mechanisch miteinander verbunden, wobei der Werkstoff des thermisch isolierenden Verbundelements vorzugsweise aus der aus Polytetrafluorethylen, Polycarbonaten, Polyurethan, Silikonen und kohlefaserverstärkten Polymeren bestehenden Gruppe ausgewählt ist.

Das thermisch isolierende Verbundelement kann in herstellungstechnisch besonders einfacher Weise die zwischen den Einzelmodulen, dem Kältepol und dem Wärmepol verbleibenden Zwischenräume in Form von Vergußmaterial ausfüllen. Die sonst bei aktiven, Mikroelektronik enthaltenden Implantaten zu fordernde hermetische Gasdichtigkeit ist bei dem vorliegendem Anwendungsfall nicht zwingend notwendig, insbesondere dann, wenn geringe Volumenanteile Wasserdampf das Seebeck-Modul intern langzeitgemäß nicht schädigen und andererseits keine toxischen Stoffe in den Körper austreten können. Das Vergußmaterial kann daher auch gut körperverträgliches Silikon sein.

In weiterer Ausgestaltung der Erfindung weist der Energiewandler eine Mehrzahl von Modulgruppen aus miteinander elektrisch in Reihe geschalteten Einzelmodulen auf, wobei diese Modulgruppen ihrerseits elektrisch parallel geschaltet sind. Durch geeignete Anordnung einer großen Zahl (im Bereich über 100.000) von miniaturisierten Seebeck-Elementen durch z.B. mikrosystemtechnische oder halbleiter-lithografische Verfahren kann durch entsprechende elektrische Serien-Parallel-Schaltung und Zusammenfassung der Einzelelemente zu Modulgruppen ein Gesamtsystem realisiert werden, das bei einer abschätzbaren Modulfläche im Bereich von 100 bis 300 cm² und einer Temperaturdifferenz von 0,5 K kontinuierlich elektrische Leistungen im Bereich von 1 bis 3 mWatt bei einer Klemmenspannung von 1 bis 2 Volt abgeben kann. Die elektrische Quellimpedanz (Innenwiderstand) kann dabei in einem Bereich von 100 bis 300 Ω liegen, so daß ein kontinuierlich arbeitendes, sensorisches Implantat, wie insbesondere Hörhilfen, energetisch versorgbar ist.

Der Kältepol kann vorteilhaft entweder selbst einen unmittelbar unter der Haut des Implantatträgers positionierbaren großflächigen Kühlkörper bilden oder mit einem solchen Kühlkörper wärmeleitend gekoppelt sein, was zu einer erhöhten Temperaturdifferenz führt.

Der Energiewandler kann grundsätzlich jede geometrische Form annehmen. Vor allem bei relativ kleinen Modulen kann er eben und flächig aufgebaut sein. Bei größeren Modulen ist insbesondere in der bevorzugten Kopfregion und dem Mastoidbereich ein gewölbtes Element vorzuziehen, das zweckmäßig nur einige mm dick ist und einem Kalottenabschnitt entspricht, der möglichst optimal der gewölbten Kopfoberfläche angepaßt ist.

Der vorliegend beschriebene thermoelektrische Energiewandler kann mit dem vorgesehenen aktiven Implantat eine Einheit bilden und dadurch ein implantationsfertiges Gesamtmodul darstellen. Hier sind insbesondere aktive, elektronische Hörimplantate zu nennen, die mit direkter elektrischer Reizung das Innenohr stimulieren (vollständig implantierbares Cochlea Implantat) (siehe zum Beispiel EP 0 076 070 und EP 0 124 930), oder mit mechanischer Stimulation des Mittel- oder Innenohres eine Teilschädigung des Innenohres rehabilitieren (Innenohrschwerhörigkeiten). Systeme der letztgenannten Art sind u.a. bekannt aus EP 0 400 630 B und EP 0 499 940 B. Der thermoelektrische Energiewandler kann auch als eigenständiges Implantat ausgeführt sein, von dessen elektrischem Ausgang, z.B. über eine geeignete, zweipolige Steckverbindung und eine adäquate elektrische Zuleitung, die elektrische Energie dem Verbraucherimplantat zugeführt wird, das nicht zwingend am gleichen Implantationsort positioniert sein muß (typisches Beispiel: thermoelektrischer Energiewandler im Mastoid, elektrische Zuleitung durch Hals, Verbraucher ist Herzschrittmacher oder sonstiger Neurostimulator im Brustraum oder Abdominalbereich).

Die Erfindung ist nachstehend unter Bezugnahme auf die beiliegenden Zeichnungen näher erläutert. Dabei zeigen:
- Fig. 1: schematisch eine Seitenansicht des Kopfes eines Patienten mit dicht unter der Haut im Mastoidbereich implantiertem, auf dem Seebeck - Effekt basierendem thermoelektrischem Energiewandler,
- Fig. 2: eine Frontansicht der Anordnung nach Fig. 1, teilweise in Schnittdarstellung,
- Fig. 3: in größerem Maßstab einen Teilausschnitt der Anordnung nach Fig. 2,
- Fig. 4: in größerem Maßstab einen Teilquerschnitt entlang der Linie IV-IV in Fig.2,
- Fig. 5: in noch größerem Maßstab eine schematische Schnittansicht eines erfindungsgemäßen Energiewandlers
- Fig. 6: eine schematische Teilansicht eines erfindungsgemäßen Energiewandlers, welche eine Serien/Parallel - Schaltung von mehreren Seebeck - Einzelmodulen erkennen läßt,
- Fig. 7: ein Blockschaltbild für eine erste Anwendung des erfindungsgemäßen Energiewandlers,
- Fig. 8: ein Blockschaltbild für eine zweite Anwendung des erfindungsgemäßen Energiewandlers,und
- Fig. 9: ein Blockschaltbild für eine dritte Anwendung des erfindungsgemäßen Energiewandlers.

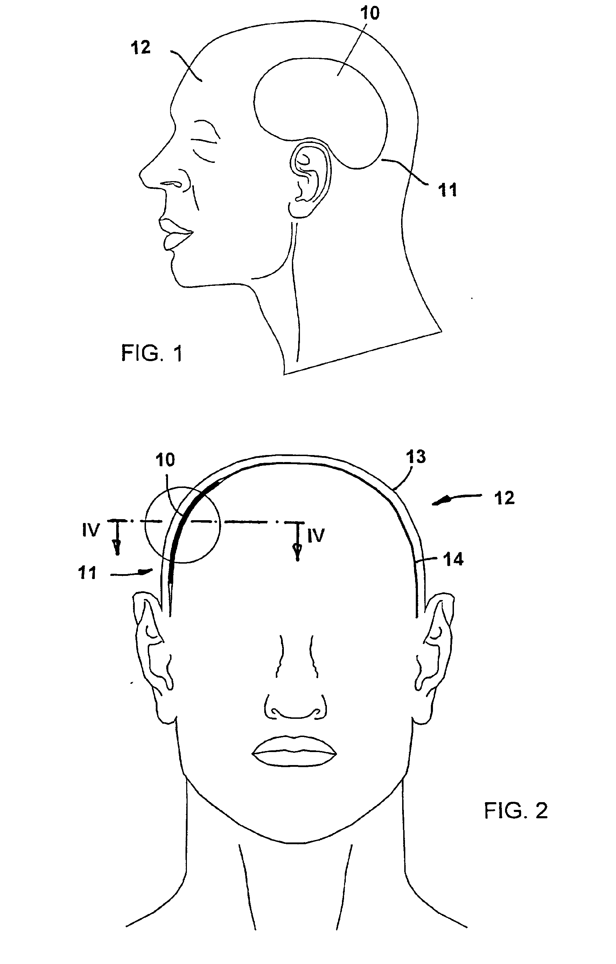

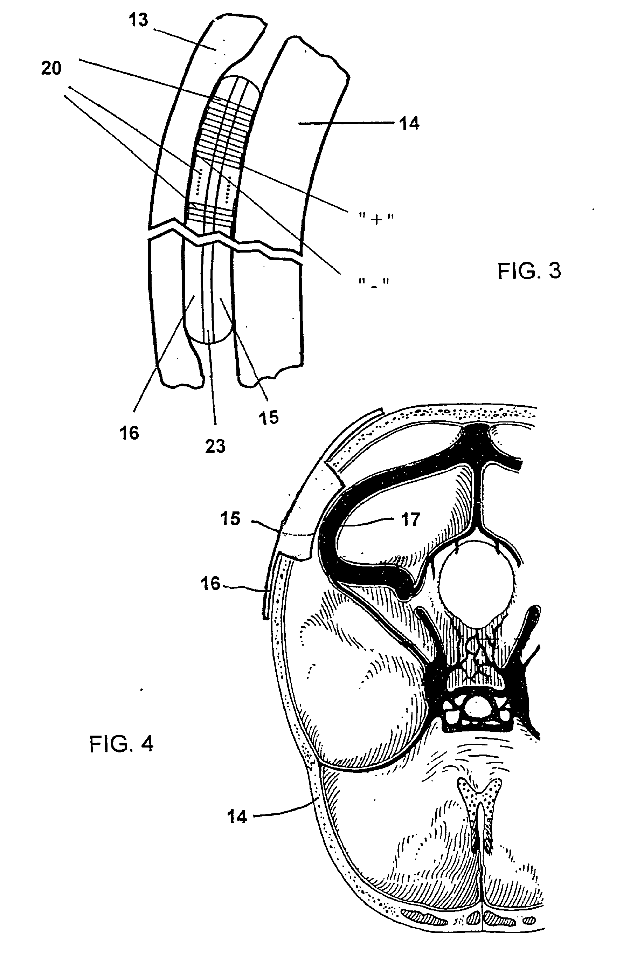

Entsprechend den Figuren 1 bis 4 ist ein auf dem Seebeck - Effekt basierender thermoelektrischer Energiewandler 10 im Mastoidbereich 11 des Kopfes 12 eines Patienten dicht unter der Haut, das heißt zwischen Kopfhaut 13 und Schädelkalotte 14, implantiert. Der Energiewandler 10 weist einen Wärmepol 15 und einen Kältepol 16 auf. Der Wärmepol 15 wird im veranschaulichten Ausführungsbeispiel mit dem Sinus Sigmoideus 17 (Fig. 4) des Implantatträgers in Wärmekopplung gehalten während der Kältepol 16 des Energiewandlers mit der benachbart dem Sinus Sigmoideus nahe der Körperoberfläche liegenden Mastoidregion 11 in Wärmekopplung gehalten wird.

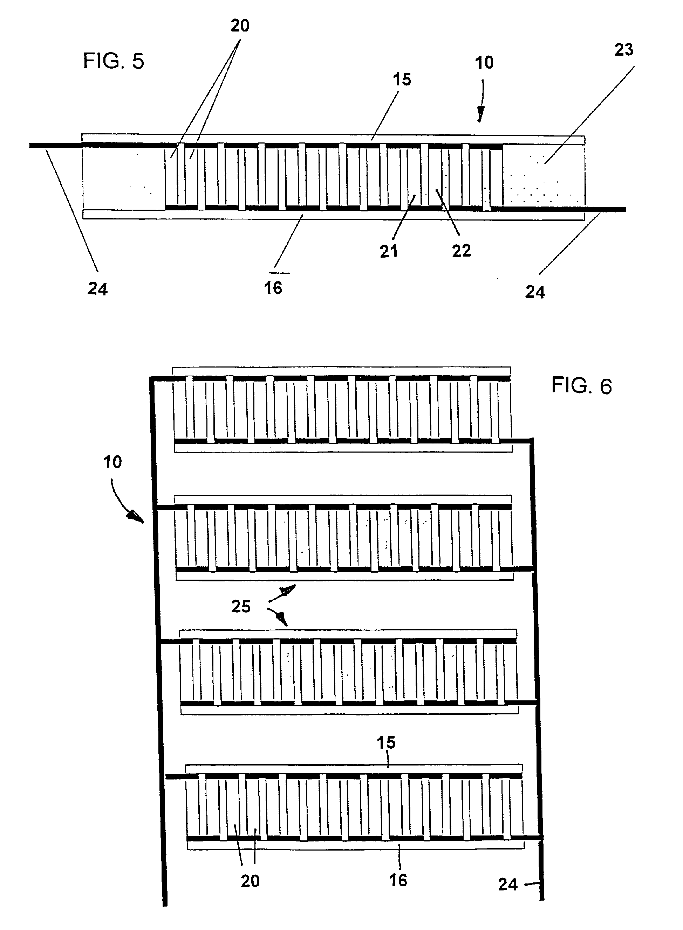

Gemäß den Figuren 3, 5 und 6 ist der Energiewandler 10 mit einer Mehrzahl *n* von miteinander elektrisch gekoppelten Seebeck - Elementen oder Einzelmodulen 20 versehen. Jedes Einzelmodul weist zwei Schenkel 21, 22 aus unterschiedlich elektrisch leitenden Werkstoffen auf, deren eines Ende mit dem Kältepol 16 und deren anderes Ende mit dem Wärmepol 15 des Energiewandlers 10 wärmeleitend verbunden ist. Die Einzelmodule 20 sind durch ein zwischen dem Kältepol 16 und dem Wärmepol 15 angeordnetes, thermisch isolierendes Verbundelement 23 aus biokompatiblem Werkstoff mechanisch miteinander verbunden. In Fig. 3 ist das thermisch isolierende Verbundelement 23 als Trennschicht zwischen Wärme- und Kältepol dargestellt. Bei der Ausführungsform gemäß Fig. 5 liegt das thermisch isolierende Verbundelement 23 in Form von Vergußmaterial vor, das die zwischen den Einzelmodulen 20, dem Kältepol 16 und dem Wärmepol 15 verbleibenden Zwischenräume ausfüllt. Die elektrischen Anschlußpole sind mit 24 bezeichnet.

Bei der Ausführungsform gemäß Fig. 6 weist der Energiewandler 10 eine Mehrzahl von Modulgruppen 25 aus miteinander elektrisch in Reihe geschalteten Einzelmodulen 20 auf Diese Modulgruppen 25 sind ihrerseits untereinander elektrisch parallel geschaltet.

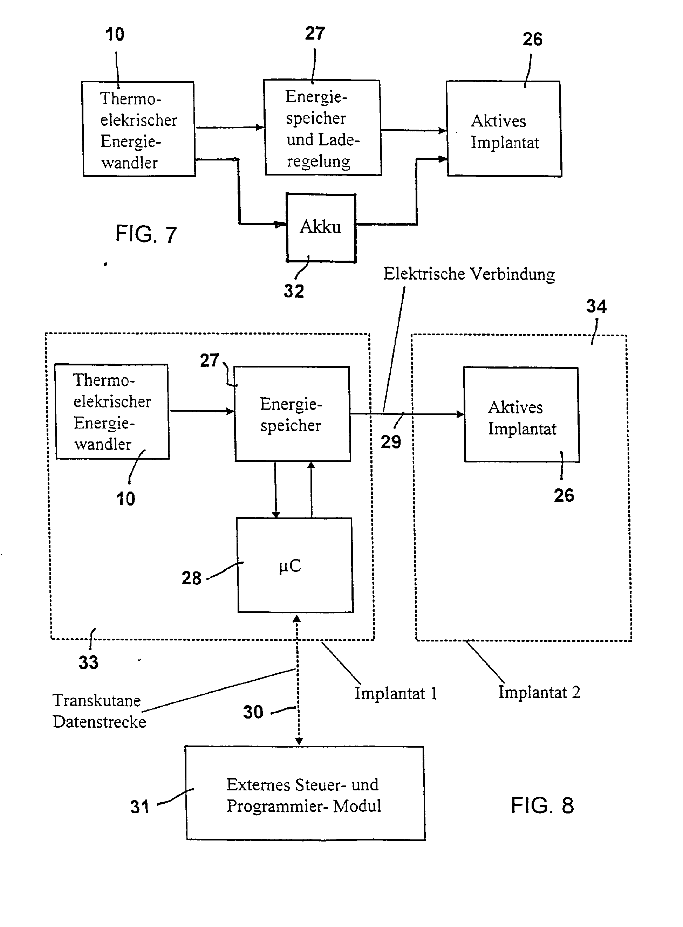

Entsprechend Fig. 7 ist zwischen den Energiewandler 10 und ein von diesem mit elektrischer Energie versorgtes teil- oder vollimplantiertes aktives Gerät (aktives Implantat) 26 ein Energiespeicher 27 geschaltet, der insbesondere aus langlebigen Kondensatoren aufgebaut und mit einem an sich bekannten elektronischen Energie - Managementsystem ausgestattet sein kann. Fakultativ kann auch ein implantierbares elektrochemisches Sekundärelement 32 (Akkumulator) vorgesehen sein, das von dem Energiewandler 10 unmittelbar oder über den Energiespeicher 27 aufgeladen wird, wenn von dem Gerät 26 nicht benötigte elektrische Energie von dem Energiewandler 10 zur Verfügung steht, und das im Bedarfsfall elektrische Energie an das Gerät 26 abgibt.

Fig. 8 zeigt eine Ausführungsform, bei der zusätzlich eine implantierbare Steuereinheit 28 vorgesehen ist, deren Aufgabe darin besteht, die Zwischenspeicherung der von dem Energiewandler 10 erzeugten elektrische Energie in der implantierten Speicheranordnung 27 und/oder die Energieweiterleitung an das Gerät 26 über eine elektrische Verbindung 29 zu regeln. Die Steuereinheit 28 kann ihrerseits mittels einer externen Steuer- und/oder Programmieranordnung 31 über eine transkutane Datenstrecke 30 von außerhalb des Körpers des Implantatträgers gesteuert und/oder programmiert werden. Bei dem Ausführungsbeispiel gemäß Fig. 8 sind der Energiewandler 10, die Speicheranordnung 27 und die Steuereinheit 28 zu einem ersten Implantat 33 zusammengefaßt, während das Gerät 26 ein zweites Implantat 34 bildet, das mit dem ersten Implantat 33 über die elektrische Verbindung 29 verbunden ist.

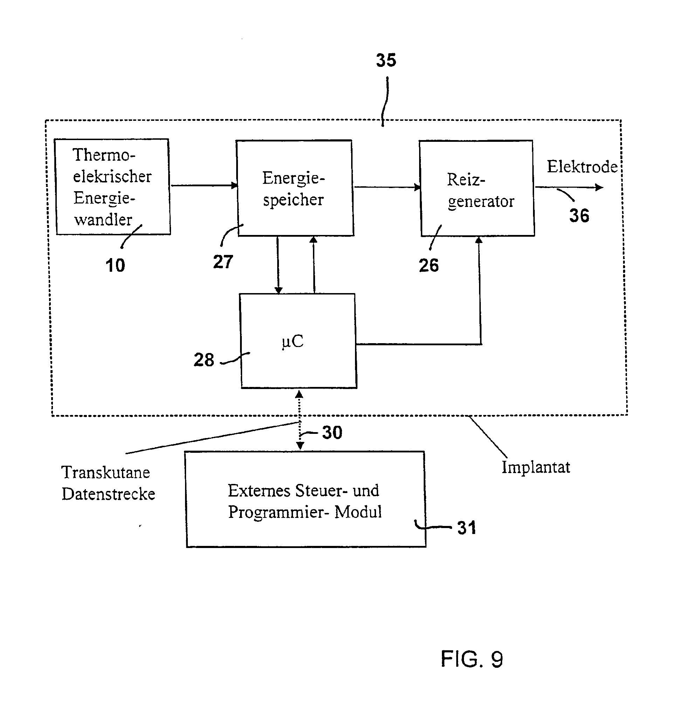

Fig. 9 zeigt dagegen ein Ausführungsbeispiel, bei dem der Energiewandler 10, die Speicheranordnung 27, die Steuereinheit 28 und das Gerät 26 in einem einzigen Implantat 35 integriert sind und bei dem mittels der Steuereinheit 28 nicht nur die Speicheranordnung 27, sondern auch das Gerät 26 gesteuert und/oder programmiert wird. Handelt es sich bei dem Gerät 26 beispielsweise um einen Reizgenerator, kann dessen Ausgang 36 mit einer entsprechenden Reizelektrode verbunden sein.

## Patentansprüche

1. Vorrichtung zur Versorgung eines mindestens teilimplantierten aktiven Gerätes (26), insbesondere einer Hörhilfe, mit elektrischer Energie, die einen implantierbaren, auf dem Seebeck - Effekt basierenden thermoelektrischen Energiewandler (10) umfasst, der die vom Körper des Implantatträgers bereitgestellte thermische Energie nutzt,
**dadurch gekennzeichnet,**
**dass** der Energiewandler (10) so geformt ist, dass er sich konform zwischen dem Knochen und der entsprechenden Haut einfügt, und eine Mehrzahl von miteinander elektrisch gekoppelten Einzelmodulen (20) aufweist,
**dass** ein Wärmepol (15) des Energiewandlers zur Wärmekopplung mit einer Wärmeenergiequelle entsprechend einem mindestens näherungsweise auf Körperkerntemperatur gehaltenen Implantationsort im Bereich der Körperoberfläche, insbesondere im Schädel- oder Halsbereich, des Implantatträgers ausgebildet ist,
**dass** ein Kältepol (16) des Energiewandlers zur Wärmekopplung mit einer Wärmesenke entsprechend einem näher an der Außenhautoberfläche, insbesondere im Schädel- oder Halsbereich, liegenden Bereich des Implantatträgers ausgebildet ist, und
**dass** jedes Einzelmodul (20) ein Ende, das mit dem Wärmepol (15) verbunden ist, und ein anderes Ende, das mit dem Kältepol (16) verbunden ist, aufweist.

2. Vorrichtung nach Anspruch 1, **dadurch gekennzeichnet, dass** der Wärmepol (15) des Energiewandlers (10) zur Wärmekopplung mit einer der großen Venen (17) oder Arterien im Schädel- oder Haisbereich des Implantatträgers ausgebildet ist.

3. Vorrichtung nach Anspruch 2, **dadurch gekennzeichnet, dass** der Wärmepol (15) des Energiewandlers (10) zur Wärmekopplung mit dem Sinus Sigmoideus (17) des Implantatträgers ausgebildet ist und dass der Kältepol (16) des Energiewandlers zur Wärmekopplung mit der benachbart dem Sinus Sigmoideus nahe der Körperoberfläche liegenden Mastoidregion (11) des Implantatträgers ausgebildet ist.

4. Vorrichtung nach einem der Ansprüche 1 bis 3, **dadurch gekennzeichnet, dass** der Kältepol (16) einen unmittelbar unter der Haut des Implantatträgers positionierbaren großsflächigen Kühlkörper bildet.

5. Vorrichtung nach einem der Ansprüche 1 bis 3, **dadurch gekennzeichnet, dass** der Kältepol (16) mit einem unter der Haut des Implantatträgers positionierbaren großflächigen Kühlkörper wärmeleitend gekoppelt ist.

6. Vorrichtung nach einem der Ansprüche 1 bis 5, **gekennzeichnet durch** eine implantierbare Speicheranordnung (27) zum Sammeln und Zwischenspeichern der von dem Energiewandler (10) erzeugten elektrischen Energie.

7. Vorrichtung nach Anspruch 6, **gekennzeichnet durch** eine implantierbare Steuereinheit (28) zum Regeln der Zwischenspeicherung der von dem Energiewandler (10) erzeugten elektrische Energie in der implantierten Speicheranordnung (27) und/oder der Energieweiterleitung an das aktive Gerät (26).

8. Vorrichtung nach Anspruch 7, **dadurch gekennzeichnet, dass** die implantierbare Steuereinheit (28) einen Mikroprozessor oder Mikrokontroller aufweist.

9. Vorrichtung nach Anspruch 7 oder 8, **gekennzeichnet durch** eine externe Steuer- und/oder Programmieranordnung (31) zum Steuern und/oder Programmieren der implantierbaren Steuereinheit (28) von außerhalb des Körpers des Implantatträgers über eine transkutane Datenstrecke (30).

10. Vorrichtung nach einem der Ansprüche 1 bis 9, **dadurch gekennzeichnet, dass** jedes Einzelmodul (20) zwei Schenkel (21, 22) aus unterschiedlich elektrisch leitenden Werkstoffen aufweist.

11. Vorrichtung nach Anspruch 10, **dadurch gekennzeichnet, dass** die Schenkel (21, 22) der Einzelmodule (20) aus Halbleiterwerkstoffen mit n- beziehungsweise p-Dotierung oder aus unterschiedlichen Metallen mit großem Abstand in der thermoelektrischen Spannungsreihe bestehen.

12. Vorrichtung nach einem der Ansprüche 1 bis 11, **dadurch gekennzeichnet, dass** der Kältepol (16) und der Wärmepol (15) aus biokompatiblem, gut wärmeleitendem Werkstoffbestehen.

13. Vorrichtung nach Anspruch 12, **dadurch gekennzeichnet, dass** der Werkstoff des Kältepols (16) und des Wärmepols (15) aus der aus reinem Titan, Titanlegierungen, Niob, Nioblegierungen, Tantal, Tantallegierungen, Edelstählen und Aluminiumoxid-Keramik bestehenden Gruppe ausgewählt ist.

14. Vorrichtung nach einem der Ansprüche 1 bis 13, **dadurch gekennzeichnet, dass** die Einzelmodule (20) durch ein zwischen dem Kältepol (16) und dem Wärmepol (15) angeordnetes, thermisch isolierendes Verbundelement (23) aus biokompatiblem Werkstoffmechanisch miteinander verbunden sind.

15. Vorrichtung nach Anspruch 14, **dadurch gekennzeichnet, dass** der Werkstoff des thermisch isolierenden Verbundelements (23) aus der aus Polytetrafluorethylen, Polycarbonaten, Polyurethan, Silikonen und kohlefaserverstärkten Polymeren bestehenden Gruppe ausgewählt ist.

16. Vorrichtung nach Anspruch 14 oder 15, **dadurch gekennzeichnet, dass** das thermisch isolierende Verbundelement (23) die zwischen den Einzelmodulen (20), dem Kältepol (16) und dem Wärmepol (15) verbleibenden Zwischenräume in Form von Vergussmaterial ausfüllt.

17. Vorrichtung nach einem der Ansprüche 1 bis 16, **dadurch gekennzeichnet, dass** der Energiewandler (10) eine Mehrzahl von Modulgruppen (25) aus miteinander elektrisch in Reihe geschalteten Einzelmodulen (20) aufweist und diese Modulgruppen ihrerseits elektrisch parallel geschaltet sind.

18. Vorrichtung nach einem der Ansprüche 1 bis 17, **dadurch gekennzeichnet, dass** der Energiewandler (10) flächig aufgebaut ist.

19. Vorrichtung nach Anspruch 18, **dadurch gekennzeichnet, dass** der Energiewandler (10) in Anpassung an die gewölbte Kopfoberfläche des Implantatträgers gewölbt ist.

20. Vorrichtung nach Anspruch 1, **dadurch gekennzeichnet, dass** eine Form des Energiewandlers (10) im wesentlichen gewölbt ist, um im Allgemeinen der gewölbten Kopfoberfläche zu entsprechen.

## Claims

1. Apparatus for supplying an at least partially implanted active device (26), in particular a hearing aid, with electric energy, which comprises an implantable, thermoelectric energy converter (10) based on the Seebeck effect, which utilises the thermal energy provided by the body of the implant wearer,
**characterised in that**
the energy converter (10) is formed in such a way that it fits comfortably between the bone and the corresponding skin, and comprises a plurality of individual modules (20) which are electrically coupled to one another,
a hot pole (15) of the energy converter is configured for thermal coupling to a thermal energy source corresponding to an implant site in the region of the body surface, in particular in the skull or neck region, of the implant wearer, which is maintained, at least approximately, at body temperature,
a cold pole (16) of the energy converter is configured for thermal coupling to a heat sink corresponding to a region of the implant wearer which is closer to the outer skin surface, in particular in the skull or neck region,
each individual module (20) comprises an end, which is connected to the hot pole (15), and another end, which is connected to the cold pole (16).

2. Apparatus according to Claim 1, **characterised in that** the hot pole (15) of the energy converter (10) is configured for thermal coupling to one of the large veins (17) or arteries in the skull or neck region of the implant wearer.

3. Apparatus according to Claim 2, **characterised in that** the hot pole (15) of the energy converter (10) is configured for thermal coupling to the sigmoid sinus (17) of the implant wearer and **in that** the cold pole (16) of the energy converter is configured for thermal coupling to the mastoid region (11) of the implant wearer adjacent to the sigmoid sinus, close to the body surface.

4. Apparatus according to any one of Claims 1 to 3, **characterised in that** the cold pole (16) forms a large-surface cooling element which can be positioned directly under the skin of the implant wearer.

5. Apparatus according to any one of Claims 1 to 3, **characterised in that** the cold pole (16) is coupled, in a heat conducting manner, to a large-surface cooling element which can be positioned under the skin of the implant wearer.

6. Apparatus according to any one of Claims 1 to 5, **characterised by** an implantable storage arrangement (27) for collecting and intermediately storing the electric energy generated by the energy converter (10).

7. Apparatus according to Claim 6, **characterised by** an implantable control unit (28) for regulating the intermediate storage of the electric energy generated by the energy converter (10) in the implanted storage arrangement (27) and/or regulating the energy transfer to the active device (26).

8. Apparatus according to Claim 7, **characterised in that** the implantable control unit (28) comprises a microprocessor or microcontroller.

9. Apparatus according to Claim 7 or 8, **characterised by** an external control and/or programming arrangement (31) for controlling and/or programming the implantable control unit (28) from outside the body of the implant wearer via a transcutaneous data link (30).

10. Apparatus according to any one of Claims 1 to 9, **characterised in that** each individual module (20) comprises two legs (21, 22) consisting of differently electrically conductive materials.

11. Apparatus according to Claim 10, **characterised in that** the legs (21, 22) of the individual module (20) consist of semiconductor materials with nor p-doping or of different metals with a large distance in the thermoelectric voltage series.

12. Apparatus according to any one of Claims 1 to 11, **characterised in that** the cold pole (16) and the hot pole (15) consist of biocompatible, good heat-conducting material.

13. Apparatus according to Claim 12, **characterised in that** the material of the cold pole (16) and of the hot pole (15) is selected from the group consisting of pure titanium, titanium alloys, niobium, niobium alloys, tantalum, tantalum alloys, stainless steels and aluminium oxide ceramic.

14. Apparatus according to any one of Claims 1 to 13, **characterised in that** the individual modules (20) are mechanically connected to each other by way of a thermally insulating composite element (23) of biocompatible material, arranged between the cold pole (16) and the hot pole (15).

15. Apparatus according to Claim 14, **characterised in that** the material of the thermally insulating composite element (23) is selected from the group consisting of polytetrafluorethylenes, polycarbonates, polyurethanes, silicons and carbon fibre reinforced polymers.

16. Apparatus according to Claim 14 or 15, **characterised in that** the thermally insulating composite element (23), in the form of potting material, fills the space remaining between the individual modules (20), the cold pole (16) and the hot pole (15).

17. Apparatus according to any one of Claims 1 to 16, **characterised in that** the energy converter (10) comprises a plurality of module groups (25) of individual modules (20) electrically connected to each other in series and these module groups are in turn connected electrically in parallel.

18. Apparatus according to any one of Claims 1 to 17, **characterised in that** the energy converter (10) is constructed in a planar manner.

19. Apparatus according to Claim 18, **characterised in that** the energy converter (10) is curved to adapt to the curved head surface of the implant wearer.

20. Apparatus according to Claim 1, **characterised in that** one form of the energy converter (10) is substantially curved in order to generally correspond to curved head surface.

## Revendications

1. Dispositif d'alimentation en énergie électrique d'un appareil actif (26) au moins partiellement implanté, en particulier une audioprothèse, qui comporte un convertisseur d'énergie thermoélectrique implantable dont le fonctionnement est basé sur l'effet Seebeck (10) et qui utilise l'énergie thermique produite par le corps du porteur de prothèse,
**caractérisé**
**en ce que** le convertisseur d'énergie (10) est constitué de telle façon qu'il peut être introduit de manière conforme entre l'os et la peau correspondante, et comporte une pluralité de modules individuels (20) accouplés électriquement entre eux,
**en ce qu'**un pôle chaud (15) du convertisseur d'énergie est constitué pour un accouplement calorifique avec une source d'énergie calorifique correspondant à un emplacement d'implantation maintenu au moins approximativement à la température corporelle interne dans la zone de la surface du corps du porteur de prothèse, en particulier dans la zone du crâne ou du cou,
**en ce qu'**un pôle froid (16) du convertisseur d'énergie est constitué pour un accouplement calorifique avec un dissipateur thermique correspondant à une zone du porteur de prothèse se trouvant plus proche de la surface externe de la peau, en particulier dans la zone du crâne ou du cou, et
**en ce que** chaque module individuel (20) comporte une extrémité qui est connectée au pôle chaud (15) et une autre extrémité qui est connectée au pôle froid (16).

2. Dispositif selon la revendication 1, **caractérisé en ce que** le pôle chaud (15) du convertisseur d'énergie (10) est constitué pour un accouplement calorifique avec une des grosses veines (17) ou artères présentes dans la zone du crâne ou du cou du porteur de prothèse.

3. Dispositif selon la revendication 2, **caractérisé en ce que** le pôle chaud (15) du convertisseur d'énergie (10) est constitué pour un accouplement calorifique avec le sinus sigmoïde (17) du porteur de prothèse et **en ce que** le pôle froid (16) du convertisseur d'énergie est constitué pour un accouplement calorifique avec la région mastoïde (11) du porteur de prothèse, située près de la surface du corps et adjacente au sinus sigmoïde.

4. Dispositif selon l'une des revendications 1 à 3, **caractérisé en ce que** le pôle froid (16) constitue un corps de refroidissement de grande surface positionnable directement sous la peau du porteur de prothèse.

5. Dispositif selon l'une des revendications 1 à 3, **caractérisé en ce que** le pôle froid (16) est accouplé thermiquement à un corps de refroidissement de grande surface positionnable sous la peau du porteur de prothèse.

6. Dispositif selon l'une des revendications 1 à 5, **caractérisé par** un ensemble de stockage implantable (27) pour collecter et stocker temporairement l'énergie électrique produite par le convertisseur d'énergie (10).

7. Dispositif selon la revendication 6, **caractérisé par** une unité de commande implantable (28) pour réguler le stockage temporaire de l'énergie électrique produite par le convertisseur d'énergie (10) dans l'ensemble de stockage implanté (27) et/ou la transmission de l'énergie à l'appareil actif (26).

8. Dispositif selon la revendication 7, **caractérisé en ce que** l'unité de commande implantable (28) comporte un microprocesseur ou un microcontrôleur.

9. Dispositif selon la revendication 7 ou 8, **caractérisé par** un ensemble externe de commande et/ou de programmation (31) pour commander et/ou programmer l'unité de commande implantable (28) depuis l'extérieur du corps du porteur de prothèse via une ligne de données transcutanée (30).

10. Dispositif selon l'une des revendications 1 à 9, **caractérisé en ce que** chaque module individuel (20) comporte deux jambes (21, 22) constituées en des matériaux qui présentent des conductivités électriques différentes.

11. Dispositif selon la revendication 10, **caractérisé en ce que** les jambes (21, 22) des modules individuels (20) sont constituées en matériaux semiconducteurs à dopage respectivement n et p, ou de métaux différents qui sont fortement éloignés entre eux dans la plage de tension thermoélectrique.

12. Dispositif selon l'une des revendications 1 à 11, **caractérisé en ce que** le pôle froid (16) et le pôle chaud (15) sont constitués en un matériau biocompatible et bon conducteur de la chaleur.

13. Dispositif selon la revendication 12, **caractérisé en ce que** le matériau du pôle froid (16) et du pôle chaud (15) est sélectionné parmi le groupe constitué par le titane pur, les alliages de titane, le niobium, les alliages de niobium, le tantale, les alliages de tantale, les aciers spéciaux et les céramiques d'alumine.

14. Dispositif selon l'une des revendications 1 à 13, **caractérisé en ce que** les modules individuels (20) sont connectés mécaniquement entre eux par un élément de connexion isolant thermique (23) agencé entre le pôle froid (16) et le pôle chaud (15) et constitué en un matériau biocompatible.

15. Dispositif selon la revendication 14, **caractérisé en ce que** le matériau de l'élément de connexion isolant thermique (23) est sélectionné parmi le groupe constitué par le polytétrafluoroéthylène, les polycarbonates, le polyuréthane, les silicones et les polymères à renfort de fibre de carbone.

16. Dispositif selon la revendication 14 ou 15, **caractérisé en ce que** l'élément de connexion isolant thermique (23) remplit les interstices restant entre les modules individuels (20), le pôle froid (16) et le pôle chaud (15) sous forme d'un matériau d'enrobage.

17. Dispositif selon l'une des revendications 1 à 16, **caractérisé en ce que** le convertisseur d'énergie (10) comporte une pluralité de groupes modulaires (25) constitués de modules individuels (20) connectés électriquement entre eux en série, et **en ce que** ces groupes modulaires sont à leur tour connectés électriquement en parallèle.

18. Dispositif selon l'une des revendications 1 à 17, **caractérisé en ce que** le convertisseur d'énergie (10) est construit de façon plane.

19. Dispositif selon la revendication 18, **caractérisé en ce que** le convertisseur d'énergie (10) est incurvé pour s'adapter à la surface incurvée du corps du porteur de prothèse,

20. Dispositif selon la revendication 1, **caractérisé en ce qu'**une forme du convertisseur d'énergie (10) est essentiellement incurvée pour correspondre généralement à la surface incurvée du corps.
